# EUROPEAN PATENT APPLICATION

(11) **EP 3 811 942 A1**
(43) Date of publication of application: **28.04.2021**
(21) Application number: 19823676.2
(22) Date of filing: 21.06.2019
(51) Int. Cl.: A61K 31/45, A61K 31/18

(54) **USE OF TYROSINE HYDROXYLASE INHIBITORS FOR THE TREATMENT OF AORTIC ANEURYSM**

(30) Priority: 21.06.2018 ES 201830607
(71) Applicant: Consejo Superior de Investigaciones Científicas (CSIC), 28006 Madrid (ES); Fundacio Institut de Recerca de l'Hospital de la Santa Creu i sant Pau, 08025 Barcelona (ES)
(72) Inventor: MARTÍNEZ GONZÁLEZ, José, 08036 Barcelona (ES); CAÑES ESTEVE, Laia, 08036 Barcelona (ES); RODRÍGUEZ SINOVAS, Cristina, 08025 Barcelona (ES)
(74) Representative: Pons
(86) International application number: PCT/ES2019/070434
(87) International publication number: WO 2019/243653

(57) **Abstract**

The present invention relates to the use of a compound that can inhibit tyrosine hydroxylase, such as alpha-methyl-p-tyrosine (AMPT), for the prevention or treatment of aortic aneurysm, preferably of the abdominal aorta.

## Description

The present invention relates to the use of a compound which is capable of inhibiting tyrosine hydroxylase for preventing or treating aortic aneurysm, preferably abdominal aortic aneurysm. Therefore, the present invention belongs to the field of pharmacology.

### BACKGROUND OF THE INVENTION

Abdominal Aortic Aneurysm (AAA) is a disease with a high rate of morbidity and mortality and a prevalence which, in men over 65, can reach 8%. In this pathology, usually asymptomatic, an irreversible degeneration of the vascular wall occurs which causes the progressive dilation of the aorta and the eventual rupture thereof (fatal in more than 80% of cases). Among the most prominent aspects of vascular remodelling in this pathology are inflammation, neovascularization, the degradation of the components of the extracellular matrix due to an increase in the activity of matrix metalloproteinases (MMPs) and death by apoptosis of vascular smooth muscle cells (VSMCs). Currently there are no pharmacological strategies that limit the development of AAA. The only therapeutic measure available is surgical intervention (open or endovascular surgery) for those aneurysms that exhibit a high risk of rupture (aortic diameter > 5.5 cm). Although it has been suggested that statins, doxycycline, COX-2 or angiotensin-converting-enzyme inhibitors, among others, could reduce the progress of AAA, none of them have conclusively demonstrated clinical benefit. Therefore, there is a need in this area to develop new pharmacological tools for treating and preventing this pathology.

The expression of the nuclear receptor NR4A3 (also known as NOR-1) is increased in coronary arteries and in the aorta with atherosclerosis of patients with ischemic heart disease. NR4A3 belongs to the NR4A nuclear receptor subfamily, which are singular nuclear receptors, since they behave as early response genes, the expression of which is rapidly induced and highly accentuated by different stimuli. These receptors regulate gene expression by binding as monomers to an NBRE element (Nerve growth Factor Responsive Element) or as dimers to an NuRE (NBRE palindromic repeat) element. Likewise, they can indirectly regulate gene expression through the modulation of signalling pathways and the activity of other transcription factors. NR4A3 modulates the migratory and proliferative response of VSMC induced by different pro-atherogenic agents such as growth factors, thrombin, native low-density lipoproteins (LDL) and oxidised LDL. NR4A3 is also critical in the proliferation and in the pro-angiogenic response induced by VEGF and thrombin in vascular endothelial cells. To exercise these functions, NR4A3 regulates the expression of genes/proteins such as cyclin D1 and SKP2 (S phase kinase-associated protein 2) which participate in cell proliferation, vitronectin which contributes to cell migration or alpha-2-macroglobulin (A2M) which regulates the activity of MMPs that participate in vascular remodelling. The role of NR4A3 in atherosclerosis is complex, since in addition to regulating the migration/proliferation and survival/apoptosis of vascular cells, it also regulates the activation and expression of adhesion molecules in endothelial cells that favor the infiltration of inflammatory cells and the lipid uptake of monocytes/macrophages. NR4A3 deficiency reduces atherosclerosis in an ApoE^{-/-} mouse model treated with a hypercholesterolemic diet. Recently, we have observed that the expression of NR4A3 is increased in human AAA and that the overexpression of the functional NR4A3 protein in the vascular wall of the aorta in mice (TgNR4A3 animals) develops aortic aneurysm after being infused with angiotensin II, without requiring a hyperlipemic genetic background and in the absence of atherosclerosis as occurs with the animal models described to date.

Today, the specific mechanism by means of which the NR4A3 receptor plays a role in the development of aortic aneurysm is unknown. The genes regulated by this nuclear receptor in the vascular wall constitute potential new therapeutic targets in aneurysmal pathology and the identification thereof is one of the objectives of the present invention in the absence of useful pharmacological strategies in this disease. It is also an object of the present invention to provide a new effective therapeutic method for treating human aortic aneurysm.

### DESCRIPTION OF THE INVENTION

The authors of the present invention have demonstrated an increase in the expression of tyrosine hydroxylase (TH) in aortic samples from patients with abdominal aortic aneurysm compared to healthy donors. Furthermore, both in the aorta of transgenic mice for NR4A3 and in mice deficient in apolipoprotein E (Apo E^{-/-}), both models susceptible to the development of aortic aneurysms, the authors detect a strong induction of TH expression in comparison with control mice resistant to the development of this pathology. TH is the limiting enzyme in the synthesis of catecholamines; it catalyses the hydroxylation of tyrosine to dihydroxyphenylalanine (L-DOPA) from which dopamine catecholamines, epinephrine and norepinephrine are synthesized. Transcription factors of the NR4A family participate in TH regulation.

The present invention relates to the use of TH inhibitors as drugs for treating aneurysmal disease and provides a method for treating, preventing, regressing or slowing down the development of human aneurysmal disease which comprises administering to the individual a sufficient amount of a TH inhibitor for reducing the vascular diameter or slowing down the dilation thereof, decreasing inflammation and/or aortic degeneration. As used herein, the terms therapy and treatment are defined in a general way that comprises the cure of the disease, or any improvement, inhibition, mitigation or control of the presence, prevalence, severity, symptoms etc. of the illness.

In a first aspect, the present invention relates to a tyrosine hydroxylase inhibitor compound for use in treating or preventing aortic aneurysm.

"Aortic aneurysm", "aorta aneurysm" or "aortic aneurysmal disease" is the disease, usually asymptomatic, that causes an abnormal widening of the aorta walls, the aorta being the main artery that runs from the heart through the chest and abdomen. In this disease there is a degeneration of the vascular wall that causes the progressive dilation of the aorta and the eventual rupture thereof. Therefore, if an aneurysm grows, it can rupture, causing dangerous bleeding and even death. Preferably, aortic aneurysm is considered to be those dilations that give rise to an increase in the external aortic diameter greater than or equal to 1.5 times with respect to the external diameter of the aorta of a healthy individual of the same species that does not present an aneurysm. The most prominent aspects of vascular remodelling that occur in the presence of aneurysmal disease are, but not limited to, inflammation, neovascularization, the degradation of the components of the extracellular matrix due to an increase in the activity of matrix metalloproteinases (MMPs) and the death by apoptosis of VSMCs. There are two types of aortic aneurysm, both included in the context of the present invention: thoracic aortic aneurysm, which occurs in the portion of the aorta that passes through the chest, and abdominal aortic aneurysm, which occurs in the portion of the aorta that passes through the abdomen.

In a preferred embodiment, said aortic aneurysm occurs in the thoracic aorta or in the abdominal aorta. In a more preferred embodiment, the aortic aneurysm occurs in the abdominal aorta.

In a preferred embodiment, said TH inhibitor compound is alpha-methyl-p-tyrosine (AMPT).

The TH inhibitor compound must be in a pharmaceutically acceptable form for therapeutic use of the invention. As it is used herein, "pharmaceutically acceptable" means suitable for use in contact with human and animal tissues without undue toxicity, irritation, allergic response and the like, consistent with a reasonable risk/benefit ratio, and effective for the intended use thereof within the scope of good medical judgment.

For the purpose of the present invention, the TH inhibitor compound may be in the form of a solvate or salts.

As it is used herein, "solvate" means a complex formed by solvation (the combination of solvent molecules with molecules or ions of the active agent of the present invention), or an aggregate consisting of a solute ion or molecule (the active agent of the present invention) with one or more solvent molecules. In the present invention, the preferred solvate is hydrate. Examples of hydrate include, but are not limited to, hemihydrate, monohydrate, dehydrate, trihydrate, hexahydrate, etc. A person skilled in the art should understand that the pharmaceutically acceptable salt of the present compound can also exist in a solvate.

The term "salts" includes derivatives of an active agent, wherein the active agent is modified by making acid or base addition salts thereof. Preferably, the salts are pharmaceutically acceptable salts. Such salts include, but are not limited to, pharmaceutically acceptable acid addition salts, pharmaceutically acceptable base addition salts, pharmaceutically acceptable metal salts, ammonium salts and alkylated ammonium salts. Acid addition salts include inorganic acid and organic acid salts. Representative examples of suitable inorganic acids include hydrochloric, hydrobromic, hydroiodic, phosphoric, sulphuric, nitric acids and the like.

In another preferred embodiment, said compound is administered in combination with at least one of other agents selected between lipid-lowering, antihypertensive, beta blocker, calcium channel blocker, diuretic, nitrate or angiotensin converting enzyme inhibitor drugs.

In another preferred embodiment, said compound is administered in a pharmaceutical composition comprising at least one biocompatible excipient.

In the present invention, "pharmaceutical composition" is understood as the composition which contains at least one excipient and one or more active agents in which at least one of them is a TH inhibitor. The TH inhibitor can inhibit the TH gene and/or protein and/or activity. Such inhibitors can be molecules or compounds of any nature (biological, synthetic/chemical or a combination of both), commercially available or molecules in development.

The composition can be administered by any route including, but not limited to, orally or parenterally, such as intravenous, intramuscular, subcutaneous, intracapsular, intraperitoneal, intrarectal, intranasal, or dermal or intradermal, by means of for example a dermal or transdermal patch. Furthermore, the administration system can be, for example, but not limited to, injection, oral, spray or inhaler. However, the administration route of a particular candidate agent will partially depend on the chemical structure thereof and can be determined by those skilled in the art. The TH inhibitor may further be combined with a pharmaceutically acceptable excipient, adjuvant and/or vehicle and formulated for the suitable administration thereof. Appropriate formulations are, but not limited to, solids (capsules, pills, caplets, tablets, etc.), semisolids (powders, granulated forms, gels or hydrogels, creams, ointments, balsams, mousses, pomades, foams, lotions, etc.) or liquids (solution, suspension, emulsion, oil, liniment, syrup, serum, vaporiser, spray, etc.), or the inhibitor can be included in a sustained release system.

The TH inhibitor compounds of the present invention can be administered orally to humans in a dosage range of 1 to 100 mg/kg of body weight in divided doses. However, it must be understood that the specific dose level and dosage frequency for any particular patient can vary and will depend on a variety of factors including the activity of the specific compound used, the metabolic stability and action duration of that compound, age, body weight, general state of health, sex, diet, mode and time of administration, excretion rate, combination of drugs, the severity of the particular condition, and the therapy the host undergoes.

Another aspect of the invention relates to the use of a TH inhibitor for manufacturing a medicament for treating aortic aneurysm. In a preferred embodiment, said inhibitor is AMPT.

Another aspect of the invention is a method for treating or preventing aortic aneurysm which comprises administering a therapeutically effective amount of a tyrosine hydroxylase inhibitor compound to a patient in need thereof. Preferably, said compound is AMPT.

"Therapeutically effective amount" of a pharmaceutical composition is understood as a therapeutically or prophylactically effective amount. A "therapeutically effective amount" relates to an effective amount, to the doses and for the necessary periods of time for achieving the desired therapeutic effect, which is an improvement in the phenotype of the aorta or any improvement, inhibition, presence mitigation or control of the prevalence, severity, symptoms etc. of the illness. The therapeutically effective amount of a compound can vary depending on factors such as the stage of the disease, subject's age, sex and weight. The dosage regime should be adjusted to provide the optimal therapeutic response. The therapeutically effective amount is also that in which any toxic or adverse effects are more than outweighed by the beneficial therapeutic effects. A "prophylactically effective" amount relates to an effective amount, to the doses and for the necessary periods of time, to achieve the desired prophylactic result, such as an improvement in the phenotype of the aorta. Usually, a prophylactic dose is used in subjects in an early stage of the disease in which the prophylactically effective amount is smaller than the therapeutically effective amount. Dose values may vary with the severity of the condition to be treated. For a particular subject, the dose regime should be adjusted over time, according to individual needs and the criteria of the professionals responsible for the administration thereof, and adjusted for providing the optimal therapeutic response.

"Improvement in the phenotype of the aorta" is understood as any phenotypic (anatomical) change in the aorta that implies an improvement with respect to the phenotype (anatomy) of the aorta in the presence of aneurysm and in the absence of the molecule or compound to be analysed. Said improvement can be, for example, but not limited to, a reduction in the diameter of the aorta, or in the inflammation parameters, neovascularization, degradation of the components of the extracellular matrix or death of VSMCs or a reduction in the severity of the pathology preferably according to the Manning scale.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration, and are not intended to limit the present invention.

### DESCRIPTION OF THE FIGURES

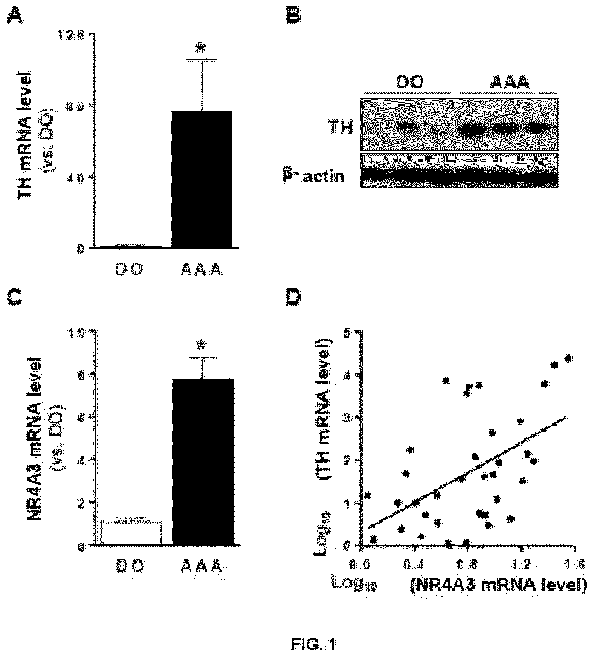

**FIG. 1****.** Expression of NR4A3 and tyrosine hydroxylase (TH) in the aorta of patients with abdominal aortic aneurysm (AAA). (A and B) TH mRNA (A) and protein (B) levels in abdominal aorta of patients with AAA. A) TH expression level in patients with AAA (n=42) and in healthy donors (n=10) analysed by means of PCR in real time. The results are expressed as the mean ± SEM relative to the expression levels in donors. *P <0.001 vs. Donors. B) TH protein levels analysed by western-blot in said samples. The image of a representative result is shown. β-actin levels were used as a load control. C) NR4A3 mRNA levels analysed by means of PCR in real-time in the samples described in (A). The results are expressed as the mean ± SEM relative to the expression levels in donors. *P <0.001 vs. Donors. D) Correlation between the TH and NR4A3 mRNA levels in said samples (r=0.4484, P< 0.005), Spearman Rank-Order Correlation).

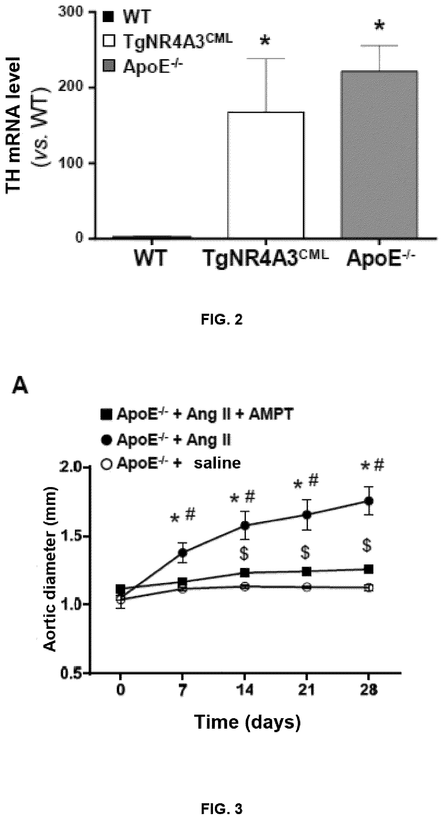

**FIG. 2****.** Expression of tyrosine hydroxylase (TH) in animal models of aortic aneurysm. TH mRNA levels analysed by means of PCR in real time in the aorta of two models susceptible to the development of aneurysm by infusion of Ang II (TgNR4A3^{CML} mice and deficient for apolipoprotein E (ApoE^{-/-})), and in C57BL/6J mice (non-transgenic control, WT) resistant to the development of AAA. Data relative to WT animals are expressed as the mean ± SEM (at least n=7). * P <0.001 vs. WT mice.

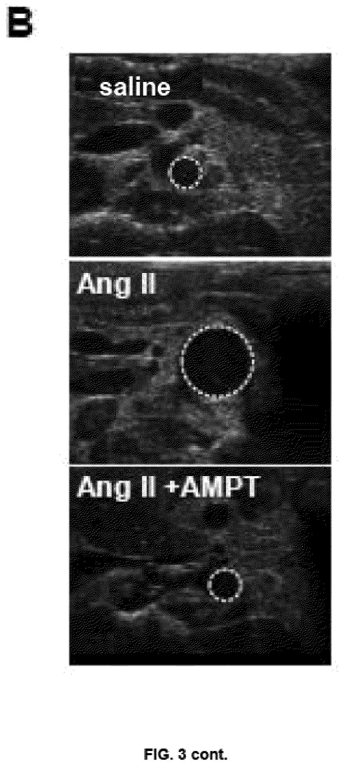

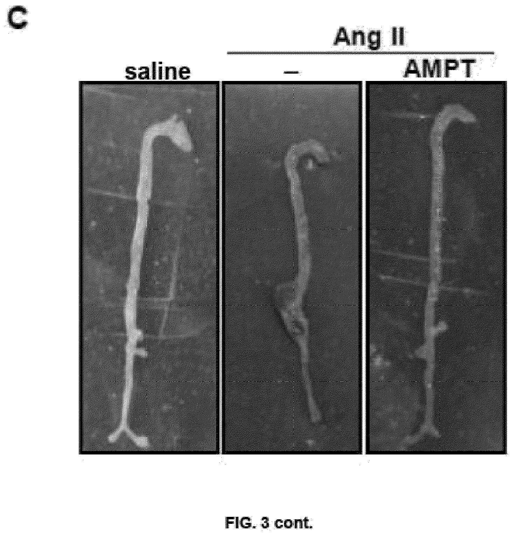

**FIG. 3****.** AMPT treatment inhibits the development of aortic aneurysm in ApoE^{-/-} mice infused with angiotensin II. (A) Graph which represents the weekly evolution of the aortic diameter of ApoE^{-/-} mice infused with saline or angiotensin II (Ang II; 1000 ng/kg/min for 28 days) analysed by means of ultrasonography. Animals infused with Ang II were treated or not with AMPT (100 mg/kg via IP, administered twice a day from the day before implementing the Ang II pump). The values correspond to the mean ± SEM (saline, n = 9; Ang II, n = 14; Ang II+AMPT, n=19). P < 0.05: * vs. time 0 of the same experimental group; # vs. animals infused with saline at the same time; $ vs. animals infused with Ang II (not treated with AMPT) at the same time. (B) Representative images obtained by means of ultrasonography of the cross-sectional view of the aorta of each of the experimental groups indicated in (A) after 4 weeks of treatment. (C) Representative photographs of the aortas of the mice in the 3 study groups at the end of the study.

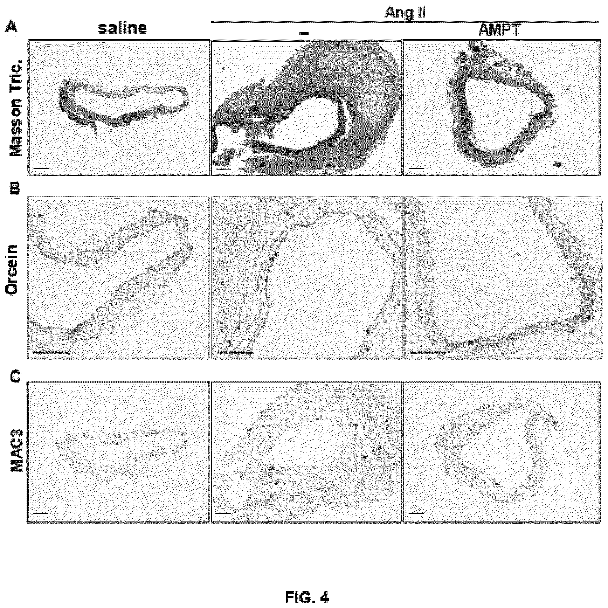

**FIG. 4****.** AMPT treatment limits structural alterations and inflammatory infiltrate in the aorta of ApoE^{-/-} mice infused with angiotensin II. ApoE^{-/-} mice were infused with saline or angiotensin II (Ang II; 1000 ng/kg/min for 28 days). Animals infused with Ang II were treated or not with AMPT (100 mg/kg, via IP, administered twice a day from the day before implementing the Ang II pump). (A and B) Representative images of the histological analysis using Masson's trichrome (A) and orcein (B) staining in sections of the abdominal aorta of the indicated study groups. Arrows indicate elastic fibre ruptures. (C) Representative staining for MAC3 (macrophage marker) in sections of the abdominal aorta of each one of the experimental groups. Arrows indicate positive regions for MAC3. Bar: 100 microns.

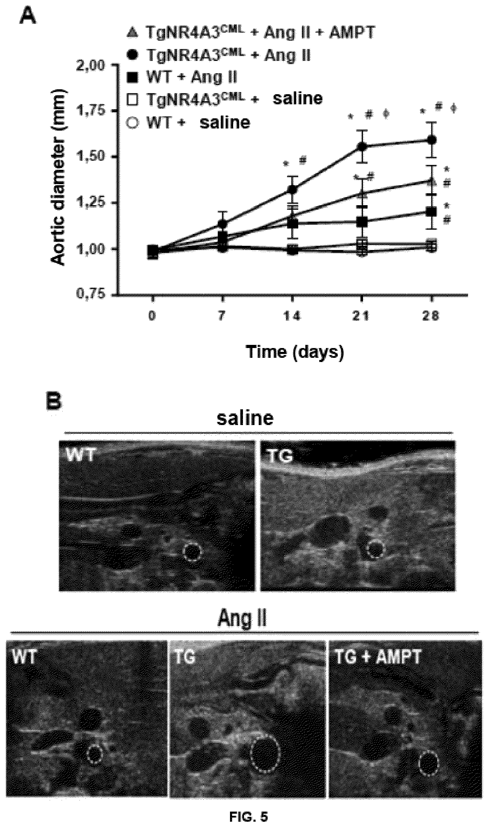

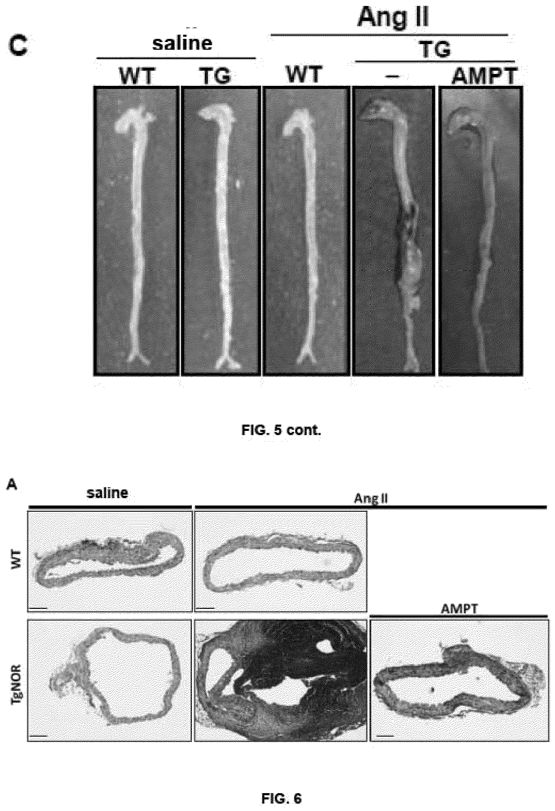

**FIG. 5** AMPT treatment inhibits the development of aortic aneurysm in TgNR4A3^{CML} mice infused with angiotensin II. (A) Diameter of the aorta of non-transgenic mice (controls; C57BL/6J; WT) and transgenic mice for human NR4A3 (TgNR4A3^{CML}) infused with saline or angiotensin II (Ang II; 1000 ng/kg/min for 28 days). A group of TgNR4A3^{CML} mice infused with Ang II was treated with AMPT (100 mg/kg, via IP, administered twice a day from the day before implementing the Ang II pump). The diameter of the aorta was determined by means of ultrasonography. Values are expressed as mean ± SEM (at least n=7). P < 0.05: * vs. time 0 of the same experimental group; # vs. the same animal infused with saline at the same time; φ vs. TgNR4A3CML infused with Ang II and treated with AMPT. (B) Representative images obtained by means of ultrasonography of the cross-sectional view of the aorta of each of the experimental groups indicated in (A) after 4 weeks of treatment. (C) Representative photographs of the aortas of the mice in the 5 groups at the end of the study.

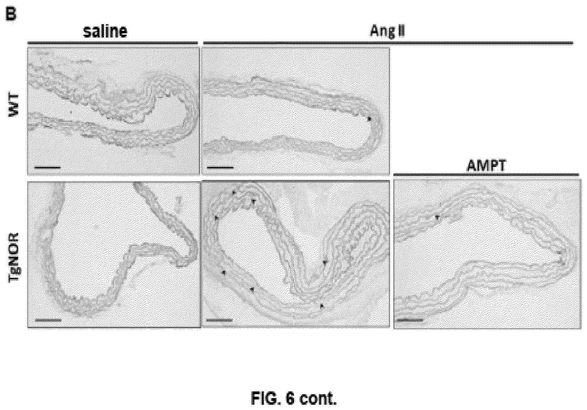

**FIG. 6****.** AMPT treatment limits structural alterations and inflammatory infiltrate in the aorta of TgNR4A3^{CML} mice infused with angiotensin II. Non-transgenic (controls; C57BL/6J; WT) and transgenic mice for human NR4A3 (TgNR4A3^{CML}) were infused with saline or angiotensin II (Ang II; 1000 ng/kg/min for 28 days). A group of transgenic animals infused with Ang II were treated or not with AMPT (100 mg/kg, via IP, administered twice a day from the day before implementing the Ang II pump). (A and B) Representative images of the histological analysis using Masson's trichrome (A) and orcein (B) staining in sections of the abdominal aorta of the indicated study groups. Arrows indicate elastic fibre ruptures. (C) Representative staining for MAC3 (macrophage marker) in sections of the abdominal aorta of each one of the experimental groups. Arrows indicate positive regions for MAC3. Bar: 100 microns.

### EXAMPLES

Next, the invention will be illustrated by means of trials carried out by the inventors, which demonstrate the effectiveness of the treatment with AMPT, a TH inhibitor, for reducing the incidence and severity of aortic aneurysms developed in response to the infusion with Ang II in NOR-1 transgenic mice and Apo E^{-/-} mice.

### Example 1. Analysis of the NR4A3 and TH expression levels in the aorta of patients with AAA and healthy donors.

Aneurysmal arterial wall samples from patients subjected to open reconstructive and abdominal aorta surgery from multiorgan donors that were immediately frozen in liquid nitrogen were obtained for subsequent analysis of mRNA and protein. NR4A3 and TH expression levels (mRNA) were analysed in said samples by means of PCR in real time. For this, total RNA was extracted from the indicated aortic samples with the RNeasy Micro kit system (Qiagen). RNA (1 µg) was reverse transcribed to cDNA by means of the High Capacity cDNA Reverse Transcription kit (Applied Biosystems) in the presence of random sequences. Likewise, for determining protein levels by Western blot, the tissue samples were homogenized in a cold lysis buffer containing 50 mM Tris-HCI pH 7.5, 1% (w/v) Triton X-100, 150 mM NaCl and 1 10 mM DTT, supplemented with protease and phosphatase inhibitors (Roche). Proteins were resolved by SDS-PAGE under reducing conditions and transferred to polyvinylidene fluoride membranes (Immobilon, Millipore). Western blot analysis was performed using antibodies against TH (ab75875, Abcam) and against β-actin (A5441, Sigma), and detection using the secondary antibody conjugated to appropriate horseradish peroxidase and the Supersignal detection system (Pierce). The homogeneity of protein load in each lane was verified by means of Ponceau staining and the determination of β-actin levels. These analyses showed an increase in the TH expression (mRNA and protein) in the aorta of patients with AAA compared to that of healthy donors (Figure 1A and B), as well as higher levels of NR4A3 mRNA in the aneurysmal aorta (Figure 1C). Furthermore, we observed the existence of a positive correlation between the NR4A3 mRNA levels and TH levels in said samples after applying the Spearman correlation coefficient on the data transformed to Log10 (Figure 1D).

### Example 2. Analysis of TH expression levels in animal models susceptible to the development of aortic aneurysm.

The studies were developed in two models that develop AAA after infusion with Ang II: transgenic male mice that overexpress the NR43A human receptor specifically in VSMC and male ApoE^{-/-} mice. A control group of non-transgenic male mice (C57BL/6J, WT), was included in these studies, animals with low susceptibility to the development of aneurysm by infusion of Ang II. Animals from the 3 study groups (non-transgenic mice, transgenic mice for NR4A3 and ApoE^{-/-}) were culled by means of terminal intraperitoneal anaesthesia with a mixture of medetomidine (1 mg/kg) and ketamine (75 mg/kg) in saline (final volume of 200 µl), aortas were immediately harvested, connective tissue and excess perivascular fat were removed, they were immediately frozen in nitrogen and stored at -80 °C until processing thereof for extracting total RNA as indicated in Example 1. The analysis of TH expression by means of PCR in real time in the aorta of these animals showed that the two models (TgNR4A3^{CML} and ApoE ^{-/-} mice) had TH mRNA levels much higher than those detected in non-transgenic mice (Figure 2). The increase in HT expression in both models was of a similar magnitude to that observed in patients with AAA versus donors (Figure 1A). Therefore, TH expression is strongly increased in those animal models susceptible to the development of AAA.

### Example 3. Analysis of the impact of TH pharmacological inhibition on the development of aneurysm induced by Ang II in ApoE^{-/-} mice.

The studies were developed in male ApoE^{-/-} mice who were acclimatised to the facility for 1 week before the study. Animals were infused with Ang II (1000 ng/kg/min) by means of osmotic minipumps (model 1004, Alzet) implemented subcutaneously in the interscapular space of the mice after anesthesia with isoflurane. The procedure lasts approximately 15 minutes per animal. Immediately after surgery, antibiotics (penicillin, 22,000 u/Kg, i.m.) and analgesics (buprenorphine 0.05 mg/Kg, i.m.) were administered for preventing infections and limiting animal discomfort, which were kept on a heating pad until they woke up after the intervention and were carefully monitored during the post-surgery period. A group of the mice infused with Ang II received AMPT treatment (100 mg/kg, via i.p) administered twice daily from the day before implementing the Ang II pump and throughout the study (28 days). The control group consisted of ApoE^{-/-} mice infused with saline. Blood pressure was assessed weekly (at 0, 7, 14, 21 and 28 days) by means of the tail plethysmography method. The diameter of the aorta was also assessed weekly by means of abdominal ultrasound using ultrasound equipment (Vevo2100 Imaging systems; Visualsonics) with a 30 MHz transducer. To do this, the mice were anesthetised by means of 1.5% isofluorane inhalation and secured in a supine position on a heating platform. After shaving the precordial region, the transducer was applied to the abdominal wall to take measurements of the abdominal aorta. Those abdominal aortas with external diameters greater than or equal to 1.5 mm were considered aneurysmal. All measurements were carried out from the images captured by using the analysis software. After 4 weeks, the animals were euthanised by means of terminal intraperitoneal anesthesia with a mixture of medetomidine (1 mg/kg) and ketamine (75 mg/kg) in saline (final volume of 200 µl), aortas were immediately harvested, they were examined to determine the presence of aneurysms and were immediately frozen in nitrogen or fixed in paraformaldehyde for macroscopic characterisation or to be embedded in paraffin and processed for histochemical studies. These analyses found that the infusion with Ang II increased blood pressure, parameter that was not significantly altered as a consequence of AMPT treatment (Table I).

**Table I. Effect of AMPT administration on systolic pressure in Apo E^{-/-} mice infused with saline or Ang II**

| Time (days) | **sal** | **Ang II** | **Ang II+AMPT** |
|---|---|---|---|
| **0** | 102.78±2.58 | 103.77±1.39 | 100.55± 1.97 |
| **7** | 104.67±5.68 | 129.05±3.84 | 133.99±4.51 |
| **14** | 100.53±1.63 | 134.66±5.04* | 136.36±4.93* |
| **21** | 103.08±4.44 | 145.59±4.49* | 143.34±4.86* |
| **28** | 103.09±2.30 | 151.58±6.60* | 154.46±5.72* |

| | | | |
|---|---|---|---|
| sal: saline. The values expressed in mm Hg correspond to the mean ± SEM, *p <0.001 *vs.* mice infused with saline | | | |

Ultrasonographic studies demonstrated that the infusion with Ang II in ApoE^{-/-} mice significantly increased the diameter of the aorta compared to control animals infused with saline, and that this effect was attenuated by AMPT treatment (Figure 3A and B). The administration of AMPT reduced the increase in aortic diameter to values similar to those of control animals, a significant effect after 14 days of treatment and that was progressively accentuated until the end of the study after 28 days, as can also be determined through macroscopic visualisation of the aorta (Figure 3C).

Histological analysis by means of Masson's trichrome staining of 5 µm sections of paraffin-embedded and fixed aortic samples, showed that in ApoE^{-/-} mice the infusion of Ang II produces significant vascular remodelling characterised by an increase in collagen deposition that is attenuated by AMPT treatment (Figure 4A). Likewise, orcein staining showed that AMPT limited the disruption and rupture of elastic fibers induced by the infusion of Ang II in these animals (Figure 4B). TH inhibition by means of AMPT treatment further prevented the significant macrophage (MAC3-positive cells) infiltrate in the vascular wall induced by the infusion of Ang II in ApoE^{-/-} mice (Figure 4C).

### Example 4. Analysis of the impact of TH pharmacological inhibition on the development of aneurysm induced by Ang II in transgenic mice for NR4A3

The studies were developed in a similar way to that described in Example 3. In this case, the effect of the infusion of Ang II on the diameter of the aorta in control animals (C57BL/6J; WT) and in transgenic mice for NR4A3 (TgNR4A3^{CML}) was analysed. The transgenic mice infused with Ang II were divided into two groups, one of which received AMPT treatment from the day before implementation of the Ang II pumps, as described in Example 3, while the second group only received the vehicle in which this drug was administered (saline). The control groups consisted of transgenic mice and non-transgenic controls infused with saline. These studies showed that the infusion with Ang II increased blood pressure in both WT animals and transgenic mice without significant differences being observed between them or as a consequence of AMPT treatment (Table II).

**Table II. Effect of AMPT treatment on systolic pressure in WT and TgNR4A3^{CML} mice infused with saline or Ang II**

| Time (days) | **WT+sal** | **TG+sal** | **WT+Ang II** | **TG+Ang II** | **TG+Ang II+AMPT** |
|---|---|---|---|---|---|
| **0** | 105.45±1.12 | 97.22±4.22 | 98.571±6.57 | 99.88±1.19 | 100.19±1.32 |
| **7** | 100.20±1.6 | 98.29±5.38 | 115.37±2.87* | 113.67±1.58* | 111,79±1.91* |
| **14** | 101.50±1.5 | 98.833±4.73 | 126.19±5.30* | 132.48±2.57* | 129.03±1.92* |
| **21** | 103.37±2.62 | 103.72±1.28 | 143.96±16.6* | 145.96±8.20* | 146.44±6.42* |
| **28** | 97.09±3.65 | 100.65±3.61 | 147.08±3.14* | 157.80±12.53* | 158.59±3.61* |

| | | | | | |
|---|---|---|---|---|---|
| WT: non-transgenic control animals; TG: TgNR4A3^{CML+} mice; sal: saline. The values expressed in mm Hg correspond to the mean ± SEM, p < 0.05, * *vs.* animals infused with saline. | | | | | |

Analysis by means of ultrasonography demonstrated that the infusion with Ang II significantly increased the diameter of the aorta of transgenic animals compared to WT animals. AMPT treatment significantly prevented this effect after 21 and 28 days (Figure 5A and B) reaching values similar to those of WT mice infused with Ang II. Attenuation of Ang II-Induced Aortic Dilation in TgNR4A3^{CML} mice treated with AMPT was also visualised at the end point after extracting and fixing the aorta (Figure 5C).

Similarly, to that described in Example 3, AMPT treatment in TgNR4A3^{CML} mice attenuated the exacerbated vascular remodelling and collagen deposition induced by Ang II in transgenic animals, as observed after Masson's trichrome staining (Figure 6A). Orcein staining confirmed that TH inhibition by means of AMPT in animals infused with Ang II limited the destructuring and rupture of the elastic laminae observed in the aorta of TgNR4A3^{CML} mice and immunohistochemical studies for MAC3 demonstrated that this drug reduced the important vascular inflammatory infiltrate triggered by Ang II in these animals.

## Claims

1. A tyrosine hydroxylase inhibitor compound for use in treating or preventing aortic aneurysm.

2. The tyrosine hydroxylase inhibitor compound for use according to claim 1, wherein said compound is alpha-methyl-p-tyrosine (AMPT).

3. The tyrosine hydroxylase inhibitor compound for use according to any of claims 1 or 2, wherein said aortic aneurysm occurs in the thoracic aorta or in the abdominal aorta.

4. The tyrosine hydroxylase inhibitor compound for use according to any of claims 1 to 3, wherein said compound is administered in combination with at least one of other agents selected between lipid-lowering, antihypertensive, beta blocker, calcium channel blocker, diuretic, nitrate or angiotensin converting enzyme inhibitor drugs.

5. The tyrosine hydroxylase inhibitor compound for use according to any of claims 1 to 4, wherein said compound is administered in a pharmaceutical composition comprising at least one biocompatible excipient.
